(19) Europäisches Patentamt
European Patent Office
Office européen des brevets

(11) Publication number: 0 038 052
B1

(12) # EUROPEAN PATENT SPECIFICATION

(45) Date of publication of patent specification: 07.03.84

(21) Application number: 81102780.4

(22) Date of filing: 10.04.81

(51) Int. Cl.³: C 07 C 21/14, C 07 C 69/145, C 07 C 17/26, C 07 C 67/11 //A01N37/02

(54) **Method for the preparation of cis-alkenyl bromide and acetate.**

(30) Priority: 14.04.80 JP 48933/80
14.04.80 JP 48934/80

(43) Date of publication of application:
21.10.81 Bulletin 81/42

(45) Publication of the grant of the patent:
07.03.84 Bulletin 84/10

(84) Designated Contracting States:
CH DE FR GB IT LI NL

(56) References cited:
GB - A - 1 228 115
GB - A - 1 504 077
US - A - 3 825 607

(73) Proprietor: Shin-Etsu Chemical Co., Ltd.
6-1, Ohtemachi 2-chome
Chiyoda-ku, Tokyo (JP)

(72) Inventor: Yamamoto, Akira
2-1-3, Minato-cho
Joetsu-shi Niigata-ken (JP)
Inventor: Ishihara, Toshinobu
2-1-3, Minato-cho
Joetsu-shi Niigata-ken (JP)
Inventor: Taguchi, Kenichi
2-1-3, Minato-cho
Joetsu-shi Niigata-ken (JP)

(74) Representative: Jaeger, Klaus, Dr.rer.nat. Dipl.-Chem. et al,
Jaeger, Grams & Pontani Patentanwälte
Bergstrasse 48 1/2
D-8035 München-Gauting (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 038 052 B1

# Method for the preparation of cis-alkenyl bromide and acetate

## Background of the Invention

The present invention relates to a method for the synthetic preparation of a cis-alkenyl bromide such as cis-dodecen-8-yl bromide and also relates to a method for the preparation of a cis-alkenyl acetate such as cis-dodecen-8-yl acetate which can be readily obtained from cis-dodecen-8-yl bromide as an intermediate.

As is well known, cis-dodecen-8-yl acetate is a sexual pheromone substance of a noxious insect called oriental fruit moth and an attempt of its use for the extermination of the noxious insect is now under development. Unfortunately, there have been proposed no economical and industrially advantageous methods for the preparation of this sexual pheronome substance.

For example, U.S. Patent specification No. 3,906,035 discloses a synthetic method for the preparation of cis-dodecen-8-yl acetate in 6 steps from propylacetylene and iodochlorohexane as the starting reactants. This method is, however, economically and industrially not feasible because of the complicated process steps including the first step which is carried out by handling liquid ammonia necessitating very elaborate facilities when production is to be undertaken in an industrial scale in addition to the economical disadvantage caused by the expensiveness of the iodochlorohexane as the starting material.

Accordingly, the primary object of the present invention has been to provide a novel and economical method for the preparation of the above mentioned sexual pheromone compound. In carrying out their investigations for the synthesis of the compound in a relatively mild conditions with high yield by use of readily available, inexpensive starting materials, the inventors have arrived at a conclusion that the most advantageous route for the synthesis of the compound is via cis-dodecen-8-yl bromide which can be prepared according to the general synthetic method for the preparation of a cis-alkenyl bromide.

## Summary of the Invention

Thus, an object of the present invention is to provide a novel and economical method for the synthetic preparation of a cis-alkenyl bromide represented by the general formula

$$RCH{=}CHCH_2CH_2(CH_2)_nBr,$$

in which R is an alkyl group having from 1 to 8 carbon atoms and n is a positive integer from 3 to 10, or, in particular, cis-dodecen-8-yl bromide of the formula

$$C_3H_7CH{=}CH(CH_2)_7Br.$$

Another object of the present invention is to provide a novel and economical method for the preparation of a cis-alkenyl acetate, in particular cis-dodecen-8-yl acetate which is readily derived from the corresponding cis-alkenyl bromide.

The method of the present invention for the synthetic preparation of the above mentioned cis-alkenyl bromide comprises reacting an $\alpha,\omega$-dibromoalkane of the formula

$$Br(CH_2)_nBr,$$

in which n has the same meaning as defined above, with a Grignard reagent derived from cis-alkenyl-3-yl chloride.

In accordance with the above described general method for the preparation of cis-alkenyl bromide, cis-dodecen-8-yl bromide is synthetically prepared by the reaction of 1,5-dibromo pentane and the Grignard reagent of cis-hepten-3-yl chloride.

Further cis-dodecen-8-yl acetate is readily obtained by the reaction of cis-dodecen-8-yl bromide with an alkali metal salt of acetic acid.

## Detailed Description of the Preferred Embodiments

As is mentioned above, cis-alkenyl bromides as the objective compounds of the present invention are promising intermediates for the synthetic preparation of various sexual pheromone substances of several noxious insects having a promising application for the extermination of the noxious insects. For example, the cis-alkenyl bromides can be readily converted into cis-alkenyl acetates of the general formula

$$RCH{=}CHCH_2CH_2(CH_2)_nOCOCH_3$$

known as a sexual pheromone compound or quasi-pheromone compound of various noxious insects, especially, of the Lepidopteras such as butterflies or moths by the treatment of the bromide with anhydrous sodium or potassium acetate. Further, the above obtained acetate can be readily converted

into the corresponding cis-alkenyl alcohol or cis-alkenyl by the hydrolysis reaction and oxidation reaction. In addition, the above mentioned intermediate compound is useful as a reactant compound for the synthesis of another sexual pheromone compound having an increased number of carbon atoms in a molecule.

As a general method for the synthetic preparation of the above mentioned sexual pheromone compounds, there has been a disclosure in Tetrahedron, volume 33 (1977), page 1845 by the steps expressed by the following reaction equations:

$$RC{\equiv}CM + X(CH_2)_mY \rightarrow RC{\equiv}C(CH_2)_mY + MX;$$

$$RC{\equiv}C(CH_2)_mY + H_2 \rightarrow RCH{=}CH(CH_2)_mY; \text{ and}$$

$$RCH{=}CH(CH_2)_mY \rightarrow CH{=}CH(CH_2)_mA,$$

in which R has the same meaning as defined above, M is an atom of lithium or sodium, X is a bromine or iodine atom, Y is a chlorine atom or a pyranylether group, A is a group expressed by the formula $-OCOCH_3$, $-OH$ or $-CHO$, and m is a positive integer from 5 to 12.

The above described general method has several disadvantages. For example, the starting compound $RC{\equiv}CM$ is relatively expensive since the compound is prepared by use of the starting materials such as butyl lithium, sodium amide, liquid ammonia, hexamethyl phosphoric triamide and the like as well as solvents in a low temperature reaction at −40°C to −20°C carried out in a pressurizable vessel. Further, the other starting reactant compound $X(CH_2)_mY$ is also relatively expensive due to the complicated procedure for the synthesis thereof while it cannot be replaced with a relatively inexpensive compound of a similar structure $Br(CH_2)_mBr$.

In the improved method of the present invention which has been completed as a result of the extensive investigations undertaken to solve the above mentioned problems in the prior art methods, the cis-alkenyl bromide of the formula.

$$RCH{=}CHCH_2CH_2(CH_2)_nBr$$

is obtained by the reaction of $\alpha,\omega$-dibromoalkane of the formula $Br(CH_2)_nBr$ with the Grignard reagent of a cis-alkenyl chloride of the formula

$$RCH{=}CHCH_2CH_2Cl.$$

A homologation reaction of this type starting with isopropyl, sec- and tert-butyl, tert-amyl, cyclohexyl and phenyl Grignard reagents is described in Journal of the American Chemical Society, volume 96, page 7102 (1974).

The above described general method for the synthesis of cis-alkenyl bromide is industrially advantageous in several aspects given below.

In the first place, one of the starting reactants $\alpha,\omega$-dibromoalkane is readily available and relatively inexpensive. In the second place, no starting materials with industrial disadvantages such as butyl lithium and sodium amide and no solvents handled with difficulties such as liquid ammonia are used in the inventive method. Further, no chilling means is necessary in carrying out the inventive method since the reac-tion temperature is not so low as −40 to −20°C as is the case in the conventional methods.

The starting reactants and the solvents used in the inventive method are all readily available and easy to handle in an industrial scale including metallic magnesium and tetrahydrofuran. No specific facilities or equipments are necessary for the reaction and the reaction can be performed at an ordinary temperature between 0 to 66°C smoothly to give the desired product in a high yield.

The most characteristic advantage of the inventive method consists in the use of the cis-alken-3-yl chloride with good availability as the main starting reactant. The alkyl group having from 1 to 8 carbon atoms denoted by the symbol R in the structural formula

$$RCH{=}CHCH_2CH_2Cl$$

for the cis-alken-3-yl chloride is exemplified by methyl, ethyl, n-propyl, n-butyl, n-hexyl and n-octyl groups. Such a cis-alken-3-yl chloride is readily obtained from alkylacetylene by the following procedure. That is, the alkylacetylene is added dropwise under agitation into a tetrahydrofuran solution of methylmagnesium chloride prepared in a conventional manner to give an alkylacetylene magnesium chloride which is reacted with ethylene oxide followed by the hydrolysis reaction to give a 3-alkyn-1-ol. This 3-akyn-1-ol is hydrogenated in the presence of a Lindlar catalyst and then chlorinated with a chlorinated agent such as thionyl choride to give the desired cis-alken-3-yl chloride in a high yield.

It should be noted in this case that, contrary to the seeming possibility, the cross coupling reaction between and alkylacetylene magnesium chloride RC≡CMgCl and an α,ω-dibromoalkane substantially does not take place. Further, the Grinnard reagent of the formula

$$RC{\equiv}CCH_2CH_2MgCl$$

cannot be obtained from the corresponding alkyn-3-yl chloride of the formula

$$RC{\equiv}CCH_2CH_2Cl.$$

In addition, the yield of the objective compound is low in the synthetic route starting with an alkene chloride having carbon atoms smaller by one in number expressed by the formula

$$RCH{=}CHCH_2Cl$$

because the cross coupling reaction is alway accompanied by the homo-coupling reaction.

The other reactant to be reacted with Grignard reagent of the cis-alken-3-yl chloride is an α,ω-dibromoalkane of the formula

$$Br(CH_2)_nBr$$

exemplified by 1,3-dibromopropane, 1,4-dibromobutane, 1,5-dibromopentane, 1,6-dibromohexane, 1,8-dibromooctane, 1,10-dibromodecane and the like. These α,ω-dibromoalkanes can be readily prepared by the reaction of a corresponding α,ω-alkanediol with hydrobromic acid in a high yield. Many of the α,ω-dibromoalkanes are commercially available and relatively inexpensive as a general-purpose reactant for the synthesis of various kinds of industrial chemicals.

The grignard reagent of the cis-alken-3-yl chloride is prepared as a tetrahydrofuran solution of the cis-alken-3-yl magnesium chloride by the reaction of the cis-alken-3-yl chloride with metallic magnesium in dry tetrahydrofuran at 40 to 66°C.

The reaction of the Grignard reagent of the cis-alken-3-yl chloride and the α,ω-dibromoalkane to give the cis-alkenyl bromide is preferably carried out in the following manner. Thus, the α,ω-dibromoalkane is dissolved in advance in tetrahydrofuran and the Grignard reagent is added dropwise into the tetrahydrofuran solution while the temperature of the reaction mixture is controlled in a range from 0 to 40°C so that the cross coupling reaction takes place between the reactants to give the desired cis-alkenyl bromide of the formula

$$RCH{=}CHCH_2CH_2(CH_2)_nBr$$

in a high yield.

In the above reaction, the molar ratio of the reactants is preferably in such a range that 1 to 2 moles of the α,ω-dibromoalkane are used per mole of the cis-alken-3-yl chloride. It is desirable that the cross coupling reaction is accelerated by using lithium copper dichloride $LiCuCl_2$ or dilithium copper tetrachloride $Li_2CuCl_4$ as a catalyst.

The above mentioned catalyst of lithium copper dichloride or dilithium copper tetrachloride is readily obtained as a solution in tetrahydrofuran by admixing lithium chloride and copper (I) chloride in tetrahydrofuran in a molar ratio of 1:1 or by admixing lithium chloride and copper (II) chloride in tetrahydrofuran in a molar ratio of 2:1, respectively. The catalyst solution thus obtained is added in advance to the tetrahydrofuran solution of the α,ω-dibromoalkane. The amount of this catalyst is preferably in such a range that from 0.001 to 0.1 mole or, more preferably, from 0.003 to 0.02 mole of the catalyst is used per mole of the Grignard reagent of the cis-alken-3-yl chloride for both lithium copper dichloride and dilithium copper tetrachloride.

In the preparation of the Grignard reagent of the cis-alken-3-yl chloride, the amount of tetrahydrofuran used as the solvent is at least equimolar to the metallic magnesium while the total amount of tetrahydrofuran used in the initial stage of the reaction should be at least 2 moles or, preferably, in the range from 3 to 20 moles per mole of the cis-alken-3-yl chloride when combined with the tetrahydrofuran used for dissolving the α,ω-dibromoalkane as the other reactant.

The reaction mixture obtained by the cross coupling reaction in the above described manner is then filtered or washed with water to remove the salt as the by-product and other insoluble matter and, after stripping and recovery of the tetrahydrofuran as the solvent, is distilled to give the desired cis-alkenyl bromide in a high yield.

The above described synthetic procedure for the cis-alkenyl bromides is, of course, suitably applied to the preparation of cis-dodecen-8-yl bromide by using cis-hepten-3-yl chloride and 1,5-dibromopentane as the cis-alken-3-yl chloride and the α,ω-dibromoalkane, respectively.

The preparation of cis-alken-3-yl chloride may be carried out in accordance with general procedure for the synthesis of the cis-alkenyl chloride. Particular reaction conditions in this case are as follows. First, propylacetylene magnesium chloride is prepared by adding propylacetylene at 30 to

60°C with agitation into a tetrahydrofuran solution of methyl magnesium chloride obtained in a conventional manner. Then, from 1.6 to 3 moles of ethylene oxide are added dropwise to the solution of propylacetylene magnesium chloride kept at 0 to 60°C per mole of propylacetylene and the resultant reaction mixture is contacted with an aqueous acid solution to be hydrolyzed followed by distillation to give 3-heptyn-1-ol in a yield of 80 to 85%.

The 3-heptyn-1-ol obtained in this manner is dissolved in n-hexane, admixed with a Lindlar catalyst in an amount from 3 to 20% by weight and reacted with hydrogen in an equimolar amount at room temperature with agitation followed by removal of the catalyst by filtration and stripping of the n-hexane to give cis-3-hepten-1-ol in an almost quantitative yield. Chlorination of this cis-3-hepten-1-ol with a chlorinating agent such as thionyl chloride gives the desired cis-hepten-3-yl chloride in a yield of 85 to 90% of the theoretical.

The cis-alkenyl bromide or, in particular, cis-dodecen-8-yl bromide is readily converted to the corresponding cis-alkenyl acetate or cis-dodecen-8-yl acetate in a yield of at least 95% in a manner as follows. Thus, the cis-alkenyl bromide or cis-dodecen-8-yl bromide is admixed with at least equimolar amount of an anhydrous acetate of an alkali metal such as sodium acetate or potassium acetate in acetic acid as a solvent and the reaction mixture is heated with agitation under reflux followed by cooling, addition of water and phase separation to obtain the organic mixture separated from the aqeuos layer, which is distilled under reduced pressure to give the desired cis-alkenyl acetate or cis-dodecen-8-yl acetate in a high yield.

The method of the present invention is now illustrated in further detail by way of examples.

Example 1

Into a reaction mixture in a reaction vessel of 1 liter capacity composed of 24.3 g of metallic magnesium and 360 g of dry tetrahydrofuran with admixture of a bit of iodine were added dropwise 2 g of ethyl bromide under agitation and then 118.5 g (1 mole) of cis-hexen-3-yl chloride were added into the reaction mixture kept at 50°C dropwise over a period of 2 hours to be reacted with the metallic magnesium. After the end of the dropwise addition of cis-hexen-3-yl chloride, the reaction mixture was further agitated for additional one hour to complete the reaction at the same temperature followed by cooling to 20°C.

The reaction mixture thus obtained containing the Grignard reagent of cis-hexen-3-yl chloride was transferred into a dropping funnel and added dropwise into a solution in a reaction vessel of 2 liter capacity prepared in advance with 293 g (1.2 moles) of 1,6-dibromohexane and 200 g of tetrahydrofuran containing 0.424 g of lithium chloride and 0.990 g of copper (I) chloride to form lithium copper dichloride $LiCuCl_2$ dissolved therein kept at a temperature of 15 to 20°C by cooling with ice under agitation over a period of about 2 hours. After the end of the dropwise addition of the Grignard reagent, the reaction mixture was warmed to 40°C and kept for 1 hour at the temperature to complete the reaction. After cooling to room temperature, the reaction mixture was filtered to remove the catalyst and the insoluble salt precipitated therein as a byproduct. Distillation of the reaction mixture under reduced pressure following removal of the tetrahydrofuran by stripping gave 195 g of a clear and colorless liquid product which was identified to be cis-dodecen-9-yl bromide. The yield was about 79% of the theoretical value.

Example 2

Into a reaction mixture in a reaction vessel of 1 liter capacity composed of 340 ml of dry tetrahydrofuran and 24.3 g of metallic magnesium with addition of a bit of iodine was blown methyl chloride gas under agitation while the temperature of the reaction mixture was kept at 40 to 50°C by cooling from outside with ice. Then 54.5 g of propylacetylene (i-pentyne) were added dropwise into the reaction mixture kept at about 50°C followed by further agitation of the mixture for additional two hours, the temperature of the mixture being kept at 50°C. Evolution of methane gas was observed during the addition of propylacetylene.

In the next place, the temperature of the reaction mixture was decreased to 10°C or below by cooling with ice and 100 g of ethylene oxide were added dropwise thereinto gradually so as that the temperature of the reaction mixture did not exceed 40°C by the heat of reaction. The reaction mixture thus obtained was poured into 1 liter of an aqueous solution containing 300 g of ammonium chloride and 100 ml of concentrated hydrochloric acid to be hydrolyzed. The organic solution taken by phase separation of the reaction mixture into organic and aqueous layers was distilled to give 76 g of 3-heptyn-1-ol corresponding to 85% yield of the theoretical value.

Into a reaction mixture prepared by dissolving 112 g of 3-heptyn-1-ol obtained in the above described manner in 100 ml of n-hexane with addition of 10 g of a Lindlar catalyst and 10 g of pyridine was blown hydrogen gas at room temperature with agitation. Introduction of hydrogen gas was continued until absorption thereof into the reaction mixture ceased about 4 hours after beginning of introduction. The reaction mixture was then filtered and distilled to remove n-hexane leaving cis-3-hepten-1-ol, which was dissolved in 250 ml methylene chloride and 111 g of triethylamine. Into the solution thus prepared and kept at a temperature of 10°C or below were added 131 g of thionyl chloride dropwise with agitation followed by further agitation of the mixture for additional 1 hour with the temperature of the reaction mixture raised to 40°C.

The thus obtained reaction mixture was poured into 500 ml of water with agitation and the organic solution taken by phase separation was washed with 5% aqueous solution of sodium hydroxide and distilled under reduced pressure to give 112 g of cis-hepten-3-yl chloride. The yield was about 85% of the theoretical value calculated on the base of 3-heptyn-1-ol.

Into a reaction mixture in a reaction vessel of 1 liter capacity composed of 24.3 g of metallic magnesium and 360 g of dry tetrahydrofuran with admixture of a bit of iodine were added dropwise 2 g of ethyl bromide with agitation and then 132.5 g (1 mole) of cis-hepten-3-yl chloride prepared in the above procedure were added dropwise over a period of 2 hours into the reaction mixture kept at 50°C. After the end of the addition of cis-hepten-3-yl chloride, the reaction mixture was further agitated for additional 1 hour with the temperature kept at 50°C to complete the reaction followed by cooling to 20°C.

The reaction mixture was then transferred into a dropping funnel and added dropwise into a mixture in a reaction vessel of 2 liter capacity prepared in advance with 264 g (1.2 moles) of 1,5-dibromopentane and 200 g of tetrahydrofuran with admixture of 0.430 g of lithium choride and 0.676 g of copper (II) chloride to form dilithium copper tetrachloride dissolved therein with agitation over a period of about 2 hours. During the dropwise addition of the above Grignard reagent, the temperature of the reaction mixture was kept at 15 to 20°C by cooling from outside with ice.

After the end of the dropwise addition of the Grignard reagent, the reaction mixture was further agitated for additional 1 hour to complete the reaction with the temperature raised to 40°C. After cooling to room temperature, the reaction mixture was filtered to remove the precipitated salt and other insoluble matter and tetrahydrofuran as the solvent was then removed by distillation. Distillation of the resultant reaction mixture under reduced pressure gave 185 g of cis-dodecen-8-yl bromide. The yield was about 75% of the theoretical value.

Example 3

Into a reaction vessel of 1 liter capacity were introduced 195 g of the cis-dodecen-9-yl bromide prepared in Example 1, 120 g of glacial acetic acid and 100 g of anhydrous sodium acetate and the reaction mixture was heated under reflux with agitation at a temperature of 164°C for 5 hours. After the end of the above reaction time and cooling to 50°C, the reaction mixture was admixed with 300 ml of water and the organic solution taken by phase separation was distilled under reduced pressure to give 169 g of cis-dodecen-9-yl acetate. The yield was about 95% of the theoretical value. This compound is known as a sexual pheromone substance of grape berry moth.

Substantially the same reaction procedure as above was repeated excepting that 185 g of cis-dodecen-8-yl bromide prepared in Example 2 were used in place of cis-dodecen-9-yl bromide to give 161 g of cis-dodecen-8-yl acetate. The yield was about 95% of the theoretical value.

The reaction of the cis-alken-3-yl chloride and an $\alpha,\omega$-dibromoalkane was carried out substantially in the same manner as in Example 2 with 1 mole of the former and 1.2 moles of the latter. The combination of the cis-alken-3-yl chloride and the $\alpha,\omega$-dibromoalkane as well as the product compound and yield thereof in % in Experiments No. 1 to No. 6 are shown in the table below.

Each of the thus prepared cis-alkenyl bromides was reacted with sodium acetate in the same manner as in Example 3 to be converted into the corresponding cis-alkenyl acetate as shown in the table together with the values of the yield in % of the theoretical. Each of these acetate compounds is known as a sexual pheromone compound of one or more of noxious insects as shown below.

Experiment No. 1: tea totrix, European corn borer and the like
Experiment No. 2: oriental fruit moth
Experiment No. 3: cabbage looper and the like
Experiment No. 4: summer fruit tortrix and the like
Experiment No. 5: diamondback moth, cabbage army worm and the like
Experiment No. 6: pink ball worm

TABLE

| Experiment No. | Cis-alken-3-yl chloride | $\alpha,\omega$-Dibromo-alkane | Cis-alkenyl bromide | | Cis-alkenyl acetate | |
|---|---|---|---|---|---|---|
| | | | Compound | Yield, % | Compound | Yield, % |
| 1 | Cis-hexen-3-yl chloride | 1,8-Dibromo-octane | Cis-tetradecen-11-yl bromide | 72 | Cis-tetradecen-11-yl acetate | 92 |
| 2 | Cis-hepten-3-yl chloride | 1,5-Dibromo-pentane | Cis-dodecen-8-yl bromide | 75 | Cis-dodecen-8-yl acetate | 95 |
| 3 | Cis-octen-3-yl chloride | 1,4-Dibromo-butane | Cis-dodecen-7-yl bromide | 74 | Cis-dodecen-7-yl acetate | 96 |
| 4 | Cis-octen-3-yl chloride | 1,6-Dibromo-hexane | Cis-tetradecen-9-yl bromide | 76 | Cis-tetradecen-9-yl acetate | 96 |
| 5 | Cis-octen-3-yl chloride | 1,8-Dibromo-octane | Cis-hexadecen-11-yl bromide | 70 | Cis-hexadecen-11-yl acetate | 96 |
| 6 | Cis-dodecen-3-yl chloride | 1,4-Dibromo-butane | Cis-hexadecen-7-yl bromide | 76 | Cis-hexadecen-7-yl acetate | 96 |

**Claims**

1. A method for the preparation of a cis-alkenyl bromide represented by the general formula

$$RCH{=}CHCH_2CH_2(CH_2)_nBr,$$

in which R is an alkyl group having from 1 to 8 carbon atoms and n is a positive integer in the range from 3 to 10, which comprises reacting a Grignard reagent of a cis-alken-3-yl chloride represented by the general formula

$$RCH{=}CHCH_2CH_2Cl,$$

in which R has the same meaning as defined above, with an $\alpha,\omega$-dibromoalkane represented by the general formula

$$Br(CH_2)_nBr,$$

in which n has the same meaning as defined above.

2. A method for the preparation of a cis-alkenyl acetate represented by the general formula

$$RCH{=}CHCH_2CH_2(CH_2)_nOCOCH_3,$$

in which R is an alkyl group having from 1 to 8 carbon atoms and n is a positive integer in the range from 3 to 10, which comprises reacting a Grignard reagent of a cis-alken-3-yl chloride represented by the general formula

$$RCH{=}CHCH_2CH_2Cl,$$

in which R has the same meaning as defined above, with an $\alpha,\omega$-dibromoalkane represented by the general formula

$$Br(CH_2)_nBr,$$

in which n has the same meaning as defined above, to form a cis-alkenyl bromide represented by the general formula

$$RCH{=}CHCH_2CH_2(CH_2)_nBr,$$

in which R and n have each the same meaning as defined above, and reacting the cis-alkenyl bromide with an alkali metal acetate.

3. The method as claimed in claim 2 wherein the reaction of the Grignard reagent of the cis-alken-3-yl chloride and the $\alpha,\omega$-dibromoalkane is carried out in tetrahydrofuran in the presence of lithium copper dichloride $LiCuCl_2$ or dilithium copper tetrachloride $Li_2CuCl_4$.

4. The method as claimed in claim 2 wherein the reaction of the Grignard reagent of the cis-alken-3-yl chloride and the $\alpha,\omega$-dibromoalkane is carried out at a temperature in the range from 0°C to 40°C.

5. The method as claimed in claim 2 wherein the reaction of the Grignard reagent of the cis-alken-3-yl chloride and the $\alpha,\omega$-dibromoalkane is carried out with from 1 to 2 moles of the $\alpha,\omega$-dibromoalkane per mole of the Grignard reagent.

6. The method as claimed in claim 3 wherein the amount of the lithium copper di-chloride or dilithium copper tetrachloride is in the range from 0.001 mole per mole of the Grignard reagent.

7. The method as claimed in claim 3 wherein the amount of tetrahydrofuran is in the range from 3 to 20 moles per mole of the Grignard reagent.

8. The method as claimed in claim 2 wherein the reaction of the cis-alkenyl bromide and the alkali metal acetate is carried out in acetic acid.

**Revendications**

1. Procédé de préparation d'un bromure de cis-alcényle de formule générale:

$$RCH{=}CHCH_2CH_2(CH_2)_nBr$$

dans laquelle R est un groupe alkyle ayant de 1 à 8 atomes de carbone et n est un nombre entier positif compris entre 3 et 10, caractérisé en ce qu'on fait réagir un réactif de Grignard obtenu à partir d'un chlorure de cis-alcène-3-yle de formule générale

$$RCH{=}CHCH_2CH_2Cl$$

dans laquelle R a la signification donnée ci-dessus, avec un $\alpha,\omega$-dibromo-alcane de formule générale

$$Br(CH_2)_nBr$$

dans laquelle n a la même signification que ci-dessus.

2. Procédé de préparation d'un acétate de cis-alcényle de formule générale

$$RCH{=}CHCH_2CH_2(CH_2)_nOCOCH_3$$

dans laquelle R est un groupe alkyle ayant de 1 à 8 atomes de carbone et n est un nombre entier positif compris entre 3 et 10, caractérisé en ce qu'on fait réagir un réactif de Grignard obtenu à partir d'un chlorure de cis-alcène-3-yle de formule générale

$$RCH{=}CHCH_2CH_2Cl$$

dans laquelle R a la même signification que ci-dessus avec un $\alpha,\omega$-dibromo-alcane de formule générale

$$Br(CH_2)_nBr$$

dans laquelle n a la même signification que ci-dessus, pour obtenir un bromure de cis-alcényle de formule générale

$$RCH{=}CHCH_2CH_2(CH_2)_nBr$$

dans laquelle R et n ont chacun la même signification que ci-dessus, et on fait réagir le bromure de cis-alcényle avec un acétate d'un métal alcalin.

3. Procédé selon la revendication 2, caractérisé en ce que la réaction entre le réactif de Grignard obtenu à partir du chlorure de cis-alcène-3-yle et l'$\alpha,\omega$-dibromo-alcane est effectuée dans du tétrahydrofuranne en présence du dichlorure de lithium-cuivre $Li$-$Cu$-$Cl_2$ ou de tétrachlorure de di-lithium-cuivre $Li_2CuCl_4$.

4. Procédé selon la revendication 2, caractérisé en ce que la réaction du réactif de Grignard obtenu à partir du chlorure de cis-alcène-3-yle et l'$\alpha,\omega$-dibromo-alcane est effectuée à une température comprise entre 0 et 40°C.

5. Procédé selon la revendication 2, caractérisé en ce que la réaction du réactif de Grignard obtenu à partir du chlorure de cis-alcène-3-yle et l'$\alpha,\omega$-dibromo-alcane est effectuée avec une quantité comprise entre 1 et 2 moles d'$\alpha,\omega$-dibromo-alcane pour une mole de réactif de Grignard.

6. Procédé selon la revendication 3, caractérisé en ce que la quantité de dichlorure de lithium-cuivre ou de tétrachlorure de di-lithium-cuivre est comprise entre 0,001 mole et 0,1 mole pour une mole de réactif de Grignard.

7. Procédé selon la revendication 3, caractérisé en ce que la quantité de tétrahydrofuranne est comprise entre 3 et 20 moles pour une mole de réactif de Grignard.

8. Procédé selon la revendication 2, caractérisé en ce que la réaction du bromure de cis-alcényle et l'acétate de métal alcalin est effectuée dans de l'acide acétique.

**Patentansprüche**

1. Verfahren zur Herstellung eines cis-Alkenylbromids der allgemeinen Formel

$$RCH{=}CHCH_2CH_2(CH_2)_nBr$$

in der R eine Alkylgruppe mit 1 bis 8 Kohlenstoffatomen ist und n eine positive ganze Zahl im Bereich von 3 bis 10 ist, wobei ein Grignard-Reagenz eines cis-Alken-3-yl-chlorids der allgemeinen Formel

$$RCH{=}CHCH_2CH_2Cl$$

in der R die vorstehend genannte Bedeutung hat, mit einem $\alpha$-$\omega$-Dibromalkan der allgemeinen Formel

$$Br(CH_2)_nBr$$

umgesetzt wird, in dem n die oben angegebene Bedeutung hat.

2. Verfahren zur Herstellung eines cis-Alkenylacetats der allgemeinen Formel

$$RCH{=}CHCH_2CH_2(CH_2)_nOCOCH_3,$$

in der R eine Alkylgruppe mit 1 bis 8 Kohlenstoffatomen und n eine positive ganze Zahl im Bereich von 3 bis 10 sind, wobei ein Grignard-Reagenz eines cis-Alken-3-yl-chlorids der allgemeinen Formel

$$RCH{=}CHCH_2CH_2Cl,$$

in der R die vorstehend genannte Bedeutung hat, mit einem $\alpha$-$\omega$-Dibromalkan der allgemeinen Formel

$$Br(CH_2)_nBr,$$

in der n die vorstehend genannte Bedeutung hat, umgesetzt wird, und zwar unter Bildung eines cis-Alkenylbromids der allgemeinen Formel

$$RCH{=}CHCH_2(CH_2)(CH_2)_nBr,$$

in der R und n die vorstehend genannte Bedeutung haben, und daß das so erhaltene cis-Alkenylbromid anschließend mit einem Alkalimetallacetat umgesetzt wird.

3. Verfahren nach Anspruch 2, dadurch gekennzeichnet, daß die Umsetzung des Grignard-Reagenz des cis-Alken-3-yl-chlorids mit dem $\alpha$-$\omega$-Dibromalkan in Gegenwart von Lithiumkupferdichlorid $LiCuCl_2$ oder Dilithiumkupfertetrachlorid $Li_2CuCl_4$ in Tetrahydrofuran durchgeführt wird.

4. Verfahren nach Anspruch 2, dadurch gekennzeichnet, daß die Umsetzung GrignardReagenz des cis-Alken-3-yl-chlorids mit dem $\alpha$-$\omega$-Dibromalkan bei einer Temperatur im Bereich von 0°C bis zu 40°C durchgeführt wird.

5. Verfahren nach Anspruch 2, dadurch gekennzeichnet, daß die Umsetzung des Grignard-Reagenz des cis-Alken-3-yl-chlorids mit dem $\alpha$-$\omega$-Dibromalkan mit 1 bis 2 mol des $\alpha$-$\omega$-Dibromalkan je Mol Gridnard-Reagenz.

6. Verfahren nach Anspruch 3, dadurch gekennzeichnet, daß der Anteil des Lithiumkupferdichlorids oder Dilithium-kupertetrachlorids im Bereich von 0,001 bis 0,1 mol je Mol Grignard-Reagenz beträgt.

7. Verfahren nach Anspruch 3, dadurch gekennzeichnet, daß der Anteil des Tetrahydrofurans im Bereich von 3 bis 20 mol je Mol Grignard-Reagenz liegt.

8. Verfahren nach Anspruch 2, dadurch gekennzeichnet, daß die Umsetzung des cis-Alkenylbromids mit dem Alkalimetallacetat in Essigsäure durchgeführt wird.